(19) Europäisches Patentamt
European Patent Office
Office européen des brevets

(11) Publication number: 0 306 335
A1

# (12) EUROPEAN PATENT APPLICATION

(21) Application number: 88308159.8

(22) Date of filing: 02.09.88

(51) Int. Cl.4: G 01 N 33/18
B 01 J 45/00

(30) Priority: 04.09.87 US 93543

(43) Date of publication of application:
08.03.89 Bulletin 89/10

(84) Designated Contracting States:
DE FR GB IT NL SE

(71) Applicant: Bradshaw, Jerald S.
1616 Oak Lane
Provo Utah 84604 (US)

Bruening, Ronald L.
1260 East 820 North
Provo Utah 84601 (US)

Izatt, Reed M.
3624 Little Rock Drive
Provo Utah 84604 (US)

Christensen, Virginia B.
128 West 3100 North
Provo Utah 84604 (US)

Alldredge, Robert Louis
9085 Marshall Court
Westminster Colorado 80020 (US)

(72) Inventor: Bradshaw, Jerald S.
1616 Oak Lane
Provo, Utah 84604 (US)

Izaat, Reed M.
3624 Little Rock Drive
Provo, Utah 84604 (US)

Bruening, Ronald L.
1260 East 820 North
Provo, Utah 84661 (US)

Christensen, James J.
Deceased
Deceased (US)

Alldredge, Robert
9085 Marshall Court
Westminster, Colorado 80020 (US)

(74) Representative: Kearney, Kevin David Nicholas et al
KILBURN & STRODE 30 John Street
London, WC1N 2DD (GB)

## (54) Analysis of ions present at low concentrations in solutions containing other ions.

(57) The invention is a process of selective and quantitatively removing and concentrating at least one selected ion present in low concentration with other ions in higher concentration in a multiple ion solution. The method comprises bringing a complexing agent for the selected ion(s) into contact with a sufficient determined quantity of said multiple ion solution to remove and concentrate the selected ion(s) from the multiple ion solution; removing the multiple ion solution, from which the complexing agent has removed the selected ions, from the complexing agent having the selected ion(s) complexed therewith, bringing the complexing agent complexed with selected ion(s) into contact with a determined quantity of receiving liquid to break the complex and remove the selected and concentrated ion(s) from the complexing agent and determining the concentration of selected ion(s) in the said receiving liquid from which the concentration of selected ion(s) in the multiple ion solution can be calculated. The metal ions in low concentration are often heavy metal ions, such as lead ions, and the process finds particularly advantageous utility in the determination of lead content in ppb in drinking water.

The preferred apparatus for carrying out the process is a column packed with silica gel or silica-bonded macrocycle as the complexing agent.

EP 0 306 335 A1

## Description

### ANALYSIS OF IONS PRESENT AT LOW CONCENTRATIONS IN SOLUTIONS CONTAINING OTHER IONS

The present invention relates to a method of selectively and quantitatively removing and concentrating at least one selected ion present in low concentration with other ions in a multiple ion solution which comprises bringing a complexing agent for the selected ion(s) into contact with a sufficient determined quantity of the said multiple ion solution to remove the selected ion(s) from the multiple ion solution and concentrate it, removing the multiple ion solution from which the complexing agent has removed the selected ion(s) from the complexing agent having the selected ion(s) complexed therewith, bringing the complexing agent complexed with selected ion(s) into contact with a determined quantity of receiving liquid to break the complex and remove the selected and concentrated ion(s) from the complexing agent therein, and determining the concentration of selected ion(s) in the said receiving liquid from which the concentration of selected ion(s) in the multiple ion solution can be calculated.

The method is particularly advantageous where the selected ion is a metal ion e.g. a heavy metal ion, e.g. lead, silver, cadmium or mercury, which occurs in drinking water, often in such low concentrations as to be incapable of analysis by presently known methods but in sufficient amounts to poison people drinking the water over a period of years.

The process is particularly useful for determining the heavy metal ion content of drinking water when it is present in ppb. It comprises flowing the drinking water in measured quantity through a column packed with silica covalently bonded to a complexing agent for the heavy metal ions, flowing a determined and smaller quantity of receiving liquid through the said column to break the complex and take the liberated ions into solution, analyzing the solution to determine the amount of e.g. percent heavy metal ion(s) therein, and calculating therefrom the concentration of heavy metal ions in the drinking water.

The invention also extends to a method of achieving the selective and quantitative removal and concentration of a selected ion or group of ions present at low concentrations from a plurality of ions in a multiple ion solution in which the other ions may be present at much higher concentrations by bringing a known amount of the solution into contact with a composition of matter comprising silica covalently bonded to a macrocyclic compound having the formula A to D.

The method is preferably carried out using as the complexing agent a macrocyclic compound which comprises silica, e.g. sand or silica gel, having a covalent bond to certain macrocyclic compounds through a hydrocarbon chain, with or without an ether oxygen linkage. These certain macrocyclic compounds have at least four $-A-CH_2-CH_2-A-$ groups in which A is selected from O, $O-CH_2$, S, $S-CH_2$, N-R and $N-R-CH_2$ in which R represents a hydrogen atom, a lower alkyl group or a benzyl group and the hydrocarbon chain has an end group of the formula

$$\begin{array}{l} CH_3 \\ \,| \\ -Si-O-\quad \text{covalently bonded to silica} \\ \,| \\ X \end{array}$$

in which X represents a lower alkyl, phenyl, benzyl, methoxy or ethoxy group or a halogen atom or an $-O-Si\equiv$ group.

Representative families of compounds contemplated by the present invention are from the class consisting of the compounds having the formulae A to D set out below wherein the macrocycle is bonded to the silica through a hydrocarbon chain with or without an ether oxygen:

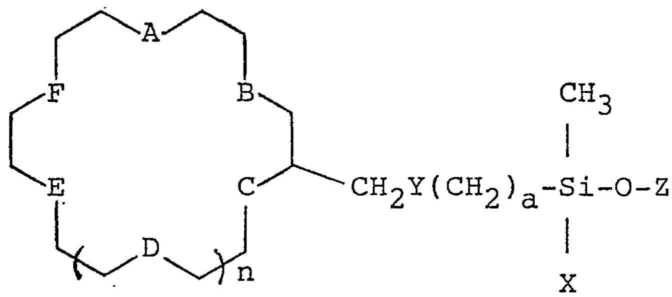

(A)

in which A to F each represent an oxygen atom or an O-$CH_2$ group or a sulphur atom or an S-$CH_2$ group or an N-R group or an N(R)$CH_2$ group in which R represents a hydrogen atom or an alkyl group, preferably a lower alkyl e.g. $C_1$-$C_4$ alkyl, or an aryl group, preferably phenylalkyl or lower alkyl, e.g. $C_1$-$C_4$, phenyl or benzyl, n is -1, 0, 1, 2, 3 or 4, X represents an alkyl group, preferably methyl or ethyl, or an aryl group, e.g. phenyl, or an alkoxy group, e.g. methoxy or ethoxy, or a halogen atom, e.g. a chlorine atom or an O-silica group or an O-silica gel group, Y represents an oxygen atom or a $CH_2$ group, a is an integer from 1 to 18, and Z represents silica or silica gel;

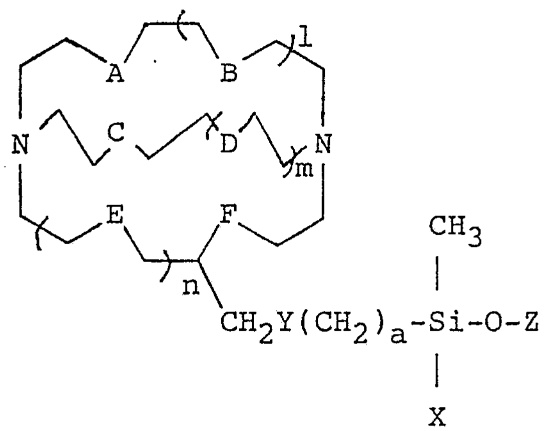

(B)

in which A to F each represent an oxygen atom or a sulphur atom or an N-R group in which R represents a hydrogen atom or an alkyl group, preferably a lower alkyl e.g. $C_1$-$C_4$ alkyl, or an aryl group preferably phenylalkyl or lower alkyl, e.g. $C_1$-$C_4$, phenyl or benzyl, l, m and n are each 0, 1 or 2, X represents an alkyl group, preferably methyl or ethyl, or an aryl group, e.g. phenyl, or an alkoxy group, e.g. methoxy or ethoxy, or a halogen atom, e.g. a chlorine atom or an O-silica group or an O-silica gel group, Y represents an oxygen atom or a $CH_2$ group, a is an integer from 1 to 18, and Z represents silica or silica gel;

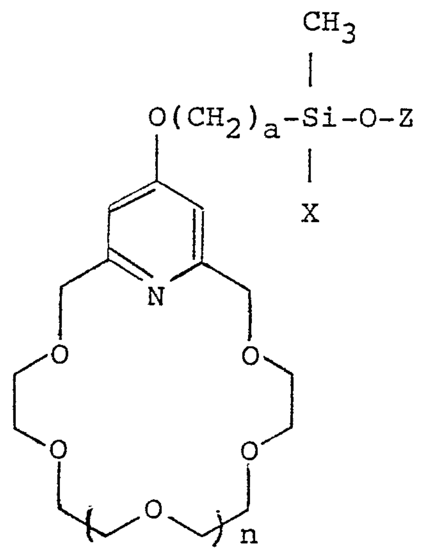

(C)

in which n is 0, 1 or 2, a is an integer from 1 to 18, X represents an alkyl group e.g. a methyl group, or an aryl group or an alkoxy group or a halogen atom e.g. a chlorine atom or an O-silica group or an O-silica gel group, and Z represents silica or silica gel; or

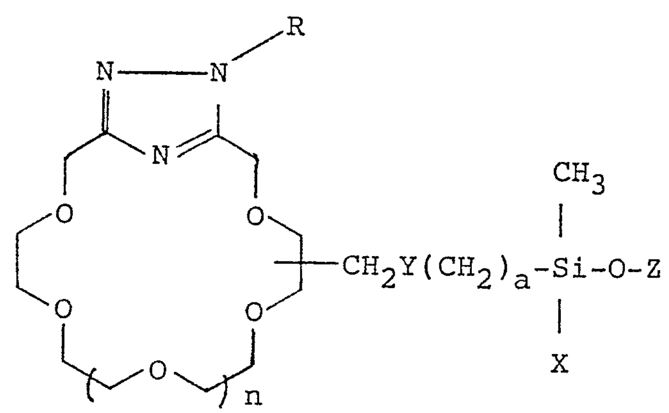

(D)

in which a is an integer from 1 to 18, Y represents an oxygen atom or a $CH_2$ group, R represents a hydrogen atom or an alkyl group or an aryl group, X represents an alkyl group e.g. a methyl group, or an aryl group or an alkoxy group or a halogen atom e.g. a chlorine atom or an O-silica group or an O-silica gel group, and Z represents silica or silica gel.

In one form of compounds A and B, R may represent a hydrogen atom or an alkyl group or a benzyl group and X may represent an alkyl group or a chlorine atom or an O-silica group, and Y represents an oxygen atom. In one form of compound C, Y represents an oxygen atom and X may represent a methyl group, a chlorine atom or an O-silica group.

In one form of compound D, Y represents an oxygen atom, R represents an alkyl group and X may represent a methyl group, a chlorine atom or an O-silica group.

A preferred embodiment disclosed herein involves carrying out the process by bringing a known volume of the multiple ion solution into contact with either a macrocycle-bonded silica or silica gel such as one of those of Formula A to D, depending on the ion(s) and solution conditions, in a separation column through which the mixture is first flowed, followed by the flow through the column of a much smaller known volume of a receiving liquid to break the complex, dissolve the desired ions and carry them out of the column. The concentrations of the desired ions can then be determined by well known analytical methods.

These compounds of formula (A) are polyether or polyazaether macrocycles (so-called crown or azacrown compounds); those of formula (B) are polyazaether macrobicycles (so-called cryptands); those of formula (C) are polypyridinoether macrocycles (so-called pyridino-crown compounds); and those of formula (D) are polytriazoloether macrocycles (so-called triazolo-crown compounds).

More particularly, a preferred embodiment of the process comprises placing either silica gel or a macrocycle-bonded silica or silica gel of Formula A to D in a tall column, causing a known volume of the mixture of ions to flow through the column where the desired ions complex with the macrocycle-bonded silica or plain silica gel which separates them from the rest of the mixture which flows out of the column, then flowing a much smaller known volume of the receiving liquid through the column to break the complex and dissolve and carry out of the column the desired ion(s). The desired ion(s) are now present in a much more concentrated form and the concentrations of the desired ion(s) present in the receiving liquid are determined by well known analytical methods.

In one preferred form of the complexing agent of formula A, A to F each represent an oxygen atom or a sulphur atom or any N-R group in which R represents a hydrogen atom or any alkyl group or any benzyl group. Thus in formula A, A, C, D and F may each represent an oxygen atom and B and E may each represent an N-R group; or in formula A or formula B, A to F may each represent an oxygen atom. In formula A, n may be 0, 1, 2, 3 or 4. In each of the formulae A to D, n may be 0, 1 or 2, and preferably a is 3. In each of the formulae, Y may represent an oxygen atom; and also X may represent a methyl group or a chlorine atom or an O-silica group or an O-silica gel group.

When the complexing agent is of formula B, l, m and n may each be 1. When the complexing agent is of formula D, R may represent any alkyl group.

In each of the formulae the said silica may be sand.

In one preferred form of complexing agent of formula A, A to F each represent an oxygen atom, n is 0, 1 or 2, a is 3, Y represents an oxygen atom, X represents a methyl group, a chlorine atom or an O-silica or an O-silica gel group and Z represents silica or silica gel; in another the only difference is that A, C, D and F each represent an oxygen atom and B and E each represent an N-R group.

In one preferred form of complexing agent of formula B, A to F each represent an oxygen atom, l, m and n are each 1, a is 3, Y represents an oxygen atom, X represents a methyl group, a chlorine atom or an O-silica gel group and Z represents silica gel.

In one preferred form of complexing agent of formula C, n is 0, 1 or 2, a is 3, Y represents an oxygen atom, X represents a methyl group or a chlorine atom or an O-silica gel group and Z represents silica gel.

In one preferred form of complexing agent of formula D, R represents an alkyl group, n is 0, 1 or 2, a is 3, Y represents an oxygen atom, X represents a methyl group or a chlorine atom or an O-silica gel group and Z represents silica gel.

The analysis of ion(s) present in water at concentrations below the 100 ppb level is generally inaccurate and/or difficult. This analysis problem is compounded when other ions are present in the same solution at much greater concentrations. Previously, we have submitted patent applications J.S. Bradshaw, R.M. Izatt and J.J. Christensen, PROTON IONIZABLE MACROCYCLIC COMPOUNDS AND SELECTIVE COMPETITIVE SEPARATION OF DESIRABLE METAL IONS FROM MIXTURES THEREOF WITH OTHER IONS, EP Appln. No. 87305221.1 filed 12th June, 1987; and J.S. Bradshaw, R.M. Izatt, J.J. Christensen, and R.L. Bruening, MACROCYCLIC LIGANDS BONDED TO SILICA AND THEIR USE IN SELECTIVELY AND QUANTITATIVELY REMOVING AND CONCENTRATING IONS PRESENT AT LOW CONCENTRATIONS FROM MIXTURES THEREOF WITH OTHER IONS, U.S. Patent Application, Serial No. 07/093544, filed 4th September, 1987, and corresponding EP Application No. which disclose the bonding of macrocycles, which do not contain electron withdrawing groups, to silica via a side chain which is not connected to one of the electron rich macrocycle donor atoms (the compounds of formulae I to IV). These bonded macrocycles have been shown to selectively form strong bonds with particular ions or groups of ions similar to the behaviour of the same macrocycles present as solutes in solution. We have also discovered in our research that plain silica gel selectively binds certain cations present as solutes in solution. Prior researchers who have studied the analytical applications of silica gel and macrocycle-bonded silicas have confined their investigations to chromatographic applications where ions are present in concentrations greater than the ppb range. The concentration and subsequent analysis of selected ions requires quantitative and selective complexation of the ions so that the ions may be sufficiently concentrated. The extent of macrocycle-ion or silica gel-cation interaction is particularly important when ions present in solution at low concentrations need to be complexed. The greater the value of the equilibrium constant for ion-macrocycle or cation-silica gel interaction, the lower the initial concentration of the ion in solution can be and still be efficiently and quantitatively complexed, and therefore removed from the solution. Silica gel forms strong bonds with only a few selected cations. However, various macrocycles form strong and selective bonds with numerous ions, when the macrocycles are present as solutes in solution. An extensive compilation of the association constants between macrocycles and various cations is found in an article by R.M. Izatt, J.S. Bradshaw, S.A. Nielson, J.D. Lamb, J.J. Christensen, and D. Sen, THERMODYNAMIC AND KINETIC DATA FOR CATION-MACROCYCLE INTERACTION, Chem. Rev., 1985, Vol. 85, 271-339. The ability to attach these macrocycles to silica without reducing the ability of the macrocycle to complex ions is of the utmost important in their use as a concentrator for analytical purposes. In this patent we report the successful use of bonded macrocycles and in certain instances plain silica gel for this purpose.

The process of recovering and concentrating the desired ion(s) is characterized by quantiatively complexing with any complexing compound, from a known volume of solution, the desired ion(s) when they are present at low concentrations and recovering the said ion(s) from the complex by bringing the complex into contact with a much smaller known volume of a receiving phase which contains a solubilizing reagent, which need not be

selective, but which will strip the ion(s) from the complex quantitatively and then analyzing the concentrated ions by well known analytical procedures.

The receiving liquid preferably contains a metal complexing agent having a higher log equilibrium constant for the ions than the said composition of matter. The ions may be analyzed by atomic absorption spectroscopy and/or ion chromatography.

The invention may be put into practice in a number of ways and a number of specific embodiments will be described by way of example to illustrate the invention with reference to the accompanying diagrammatic drawing which represents schematically a suitable column for holding macrocycle-bonded silica material through which a solution of metal ions can be flowed to complex selectively with a desired ion or group of ions and through which a small volume of a receiving phase can be flowed to break the complex, dissolve the desired ions and carry them out of the column.

Materials which selectively complex with the desired metal ions preferably are covalently bonded to silica. The silica material can be sand, silica particles or silica gel. Compounds of the formula A, B, C and D have been already disclosed and claimed by us in U.S. Patent Application, Serial No. 07/093544 by J.S. Bradshaw, R.M. Izatt, J.J. Christensen, and R.L. Bruening, MACROCYCLIC LIGANDS BONDED TO SILICA AND THEIR USE IN SELECTIVELY AND QUANTITATIVELY REMOVING AND CONCENTRATING IONS PRESENT AT LOW CONCENTRATIONS FROM MIXTURES THEREOF WITH OTHER IONS, filed 20th July, 1987. These compounds are examples of the many different types of cation complexing agents that can be used to concentrate the desired ions. Any material which complexes with the desired ions with a sufficient association constant can be used to concentrate the desired ions, preferably one which can be covalently bonded to silica.

The present patent does not claim the synthesis of these various silica-bound ion complexing agents, some of which are claimed in the aforementioned patent application. The present application claims the process of using these materials for the concentration and subsequent analysis of various ions in the ppb range. This will now be described in more detail in the following description of the processes of ion concentration, recovery, and analysis.

The ion recovery and concentration processes of the present invention relate to the selective recovery of desired ions from mixtures thereof with other ions, preferably using macrocycle-bonded silica or, in a few cases, plain silica gel. The ion(s) involved may be any cation or anion which can be quantitatively complexed with a complexing agent. The synthesis of macrocycle-bonded silica is not part of the present invention as described in the previous section of this application. Effective analysis of ions present in water at concentrations below the ppb level is difficult using present analytical methods. The need for quick and accurate analysis of toxic ions at these low concentrations is particularly important in the monitoring of these ions in culinary and waste water. Toxic ions such as $NO_3^-$, $CN^-$, $Pb^{2+}$, $Hg^{+2}$, $Cd^{2+}$, $Ag^+$, $Ba^{2+}$, (which we will call Class A ions) and others need to be monitored at the ppb level. Other less toxic ions such as $Na^+$, $K^+$, $Ca^{2+}$, $Mg^{2+}$, $SO_4^{2-}$, and $Cl^-$ (which we will call Class B ions) are also present in these solutions. Some of these latter ions, such as $Na^+$, $Ca^{2+}$, $Mg^{2+}$, etc., are present in the tens of ppm range. Hence, the need to selectively recover and concentrate the above mentioned Class A toxic ions from the Class B ions for analysis of the Class A ions. Other situations where analyses in the ppb range must be made also exist. The present invention accomplishes the necessary separation and concentration effectively and rapidly, particularly by the use of macrocycle-bonded silica or in a few cases plain silica gel so that these analyses can take place.

The process first involves selecting a complexing agent, e.g. a macrocycle-bonded silica or plain silica gel which will selectively and quantitatively complex the ion(s) of interest and thereby remove it from a solution which is brought into contact with the complexing agent, e.g. the macrocycle-bonded silica. There is a large data base for measurements of macrocycle-ion interactions where the macrocycle is unsubstituted and present as a solute in a solvent. Such a data base was compiled by R.M. Izatt, J.S. Bradshaw, S.A. Nielsen, J.D. Lamb, J.J. Christensen, and D. Sen, THERMODYANMIC AND KINETIC DATA FOR CATION-MACROCYCLE INTERACTION, Chem. Rev., 1985, Vol. 85, 271-339. Previously, this data base has only provided general predictions of the behaviour of macrocycles incorporated into separation processes in solution. However, we reported recently in U.S. Patent Application, Serial No. 07/093544, by J.S. Bradshaw, R.M. Izatt, J.J. Christensen, and R.L. Bruening, MACROCYCLE LIGANDS BONDED TO SILICA AND THEIR USE IN SELECTIVELY AND QUANTITATIVELY REMOVING AND CONCENTRATING IONS PRESENT AT LOW CONCENTRATIONS FROM MIXTURES THEREOF WITH OTHER IONS, filed 4th September, 1987, that the equilibrium constants for ion-macrocycle interaction for macrocycles present as solutes in solution vs. that for macrocycles bonded to silica show little or no variation. It is emphasised that similar interaction of the bonded macrocycle and macrocycle in solution is only obtained when electron withdrawing groups are not attached to the macrocycle and when the macrocycle is not attached to silica via ore of the donor atoms of the macrocycle. We have also made extensive measurements of the interaction of plain silica gel with cations and have found that a few particular cations do interact quantitatively and selectively with silica gel when these cations are present in the ppb range.

Once the desired ion(s) are attached to the complexing agent, e.g. the macrocycle-bonded silica, they must be removed using a volume of a receiving phase much smaller than the original volume of solution containing the desired ion(s). Furthermore, the volume of both the original source phase and the receiving phase solutions must be known in order for the concentration factor to be calculated under these circumstances. The concentrations of the ion(s) of interest present in concentrated form in the receiving phase can be easily determined by well known analytical methods such as atomic absorption spectroscopy. The concentrations of

the ion(s) of interest in the original source phase solution can then be calculated by dividing the measured receiving phase concentration by the concentration factor calculated from the relative volumes.

Examples of the successful use of the present invention to obtain accurate analyses of ion concentrations in the ppb level for solutions prepared at known concentrations will now be given.

## Example 1

In Table 1, the analysis of $Sr^{2+}$ concentrations in $H_2O$ in the ppb range using 18-crown-6 bonded to silica gel (see Formula A.1 below) is presented.

This silica-gel bonded macrocycle 18-crown-6 of formula A.1 is the compound of formula A in which A-F = oxygen, n = 1, a = 3, y = 0, Z = Silica-gel and X = $CH_3$.

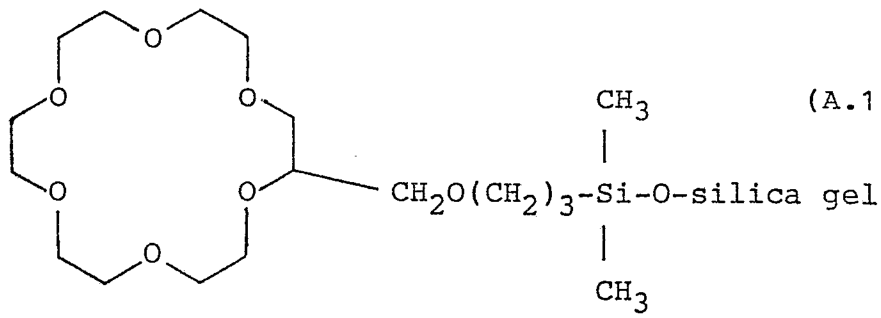

(A.1)

The analysis was tested by running volumes of known $Sr^{2+}$ concentrations through a column, concentrating the $Sr^{2+}$, and analyzing the resulting solution for $Sr^{2+}$. These volumes also contained $Ca^{2+}$, $Mg^{2+}$, and $Na^{+}$ at concentrations present in a typical culinary water supply (see footnote b to Table 1). The 18-crown-6 macrocycle was chosen because of its selective interaction with $Sr^{2+}$ over $Ca^{2+}$, $Mg^{2+}$, and $Na^{+}$ which are also present in the solution. The log of the equilibrium constant for 18-crown-6-$Sr^{2+}$ interaction is 2.72. This value is not sufficiently large for quantitative removal of $Sr^{2+}$ from a large volume of solution using the small column described above, but quantitative $Sr^{2+}$ removal by this macrocycle from a one litre volume takes place. Hence, macrocycles which selectively interact with particular ion(s) over other ions can be used in the concentration procedure even though their interaction constants may not be large. The receiving phase used to remove the $Sr^{2+}$ in a small volume was one which has a higher log equilibrium constant for the ion than the macrocycle, in this case ethylenediaminetetraacetic acid (EDTA). Instead of EDTA, citrate or acetate ions can be used. The $Sr^{2+}$ interacts more strongly with EDTA(log K=8.73) than it does with the macrocycle.

TABLE 1

Analysis of $Sr^{2+}$ Concentrations in the ppb Range using an 18-Crown-6 bonded Silica Gel Column[a] when $Ca^{2+}$, $Mg^{2+}$, and $Na^{+}$,[b] are also present in Solution

| Known Concentration[c] (ppb) | Measured Concentration[d] (ppb) |
|---|---|
| 100 | 101 ± 5 |
| 10 | 9.3 ± 0.5 |
| 1 | 0.90 ± 0.12 |

Notes on Table 1

a) The cylindrical column used was 1.90 cm in diameter and contained a 1.5 cm height of the material. The gel capacity was 5.3 moles of macrocycle/$m^3$ silica gel.

b) The cations were present as the $NO_3^-$ salts and the concentrations of $Ca^{2+}$, $Mg^{2+}$, and $Na^+$ were 52 ppm, 15 ppm, and 148 ppm, respectively.

c) The solutions were prepared at known $Sr^{2+}$ concentrations by diluting atomic absorption spectroscopy standards of known concentrations to the indicated concentrations using volumetric pippettes. Any $Sr^{2+}$ present in the $Ca^{2+}$, $Mg^{2+}$, and $Na^+$ salts was removed by passing these salts in solution through an 18-crown-6 bonded silica (see Formula A.1) column before the indicated amount of $Sr^{2+}$ was added.

d) The volume of the original sample required for analysis is 100 ml, 100 ml and 1000 ml for the 100, 10, and 1 ppb levels, respectively. A solution containing 0.03 M EDTA and 0.10 M LiOH or NaOH was used as the receiving phase.

Furthermore, the EDTA was present at a greater concentration than the macrocycle. The combination of these two effects allows for the $Sr^{2+}$ to be stripped from the macrocycle in a relatively small volume. Other materials such as citric acid and acetic acid can also be used for the removal of $Sr^{2+}$ from the column. The $Sr^{2+}$ in the receiving phase was then analyzed using atomic absorption spectrophotometry, although other analytical methods could have been used. Experiments identical to those in Table 1, but using a plain silica gel column for $Sr^{2+}$ concentration and analysis were also tried. The silica gel did not quantiatively remove the $Sr^{2+}$ from the aqueous solutions under these conditions.

The data base for ion-macrocycle interactions, mentioned above, allows for the judicious choice of an appropriate macrocycle for a specific analytical need.

Example 2

$Pb^{2+}$, $Cd^{2+}$, $Ag^+$, and $Hg^{2+}$ need to be analyzed at the ppb level in culinary water. The macrocycle diaza-18-crown-6 (see Formula A.2) interacts strongly with these cations. Furthermore, this macrocycle interacts very weakly with $Ca^{2+}$, $Mg^{2+}$, $Na^+$, and $K^+$, which are the cationic species present in water in large quantities. This macrocycle could then be used to selectively and quantitatively complex $Pb^{2+}$, $Cd^{2+}$, $Ag^+$ and $Hg^{2+}$ from a culinary water sample. The diazacrown-bonded silica has the formula A.2 given below; it is a compound of formula A in which A, C, D and F = 0, B and F = NR, R = a hydrogen atom, any alkyl group or any benzyl group, Y = 0 or $CH_2$, n = 0 -2, a = 1-16, Z = silica and X = $CH_3$, Cl or O-silica

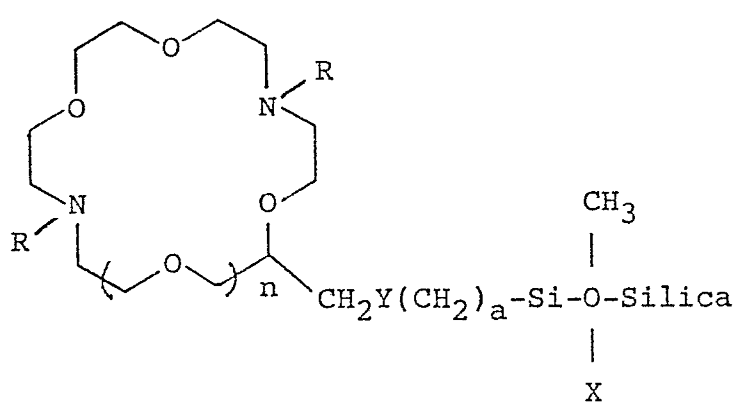

(A.2)

Specific examples of the formula A.2 which have been made and utilized are A.2.1 in which R = $C_2H_5$, n = 1, a = 3, Y = 0, X = $CH_3$ and Z = silica gel and A.2.2 in which R = $C_6H_5$, n = 1, a = 3, T = 0, X = $CH_3$ and Z = silica gel.

The chemical EDTA interacts more strongly with $Pb^{2+}$, $Cd^{2+}$, $Ag^+$, and $Hg^{2+}$ than does the macrocycle. Hence, a concentrated EDTA solution could be used as the receiving phase. Finally, an inductively coupled plasma atomic absorption spectrophotometer could be used to analyze the concentrated receiving phase for these cations all at the same time. Other macrocycles interact selectively with various other ions. Many of these macrocycles are protonionizable which allows for the use of an acid solution as the receiving phase.

Example 3

Plain silica gel interacts with sufficient strength to quantitatively bind several cations when other cations are not present in excess concentrations. The determination of $Pb^{2+}$ and $Sr^{2+}$ concentrations in the ppb range has been accomplished with standard deviations similar to those of Table 1 when any other cation(s) are present in the sample at or below the ppb level. However, plain silica gel interacts selectively with only few cations. Hence, analysis at the ppb level for a particular cation when other cations are present at much higher concentrations is possible in only a few cases. An example of this is given in Table 2 for the analysis of $Pb^{2+}$ in

the ppb range when $Ca^{2+}$, $Mg^{2+}$, and $Na^{+}$ are present in large excess. The $Pb^{2+}$ experiments were performed by the same method described above for $Sr^{2+}$ except that plain silica gel was used as the column material.

TABLE 2

Analysis of $Pb^{2+}$ Concentrations in the ppb range using a Plain Silica Gel Column [a] when $Ca^{2+}$, $Mg^{2+}$, and $Na^{+}$,[b] are also present in solution

| Known Concentration[c] (ppb) | Measured Concentration[d] (ppb) |
|---|---|
| 100 | 97 $\pm$ 11 |
| 10 | 9.0 $\pm$ 0.7 |

Notes on Table 2

a) The cylindrical column used was 1.90 cm in diameter and contained a 2.5 cm height of the material.

b) The cations were present as the $NO_3^-$ salt and the concentrations of $Ca^{2+}$, $Mg^{2+}$, and $Na^{+}$ were 52 ppm, 15 ppm, and 148 ppm, respectively.

c) The solutions were prepared at known $Pb^{2+}$ concentrations by diluting atomic absorption spectroscopy standards of known concentrations to the indicated concentrations using volumetric pippettes. Any $Pb^{2+}$ present in the $Ca^{2+}$, $Mg^{2+}$, and $Na^{+}$ salts was removed by passing these salts in solution through an 18-crown-6 bonded silica (see Formula A.1) column before the indicated amount of $Pb^{2+}$ was added.

d) The volume of the original sample required for analysis is 100 ml and 1000 ml for the 100 and 10 ppb levels, respectively. A solution containing 0.03 M EDTA and 0.10 M LiOH or NaOH was used as the receiving phase.

Example 4

Additional $Pb^{2+}$ experiments were performed using 18-crown-6 bonded silica (see Formula A.1) as the column material. The analysis data for $Pb^{2+}$ using the macrocycle-containing material is comparable to those present in Table 2. This is to be expected since both silica gel and 18-crown-6 interact selectively with $Pb^{2+}$ over $Mg^{2+}$, $Ca^{2+}$, and Ma+.

The detection limits of the processes described above is dependent on the concentration factor (volume of the source solution divided by the volume of the receiving solution) and the detection limit of the analytical method used. For example, the detection limits for $Sr^{2+}$ and $Pb^{2+}$ are 0.2 $\pm$ 0.2 ppb and 0.5 $\pm$ 0.5 ppb, respectively, if the concentration factor is 100 and inductively coupled plasma atomic absorption spectroscopy is the analytical method used.

In conclusion, many ions can be analysed in the ppb range by concentrating the ions of interest using a complexing agent, e.g. a macrocycle-bonded silica or in a few cases plain silica gel followed by the recovery of the ions in a smaller volume of a receiving phase. This process is effective even when other ions are present in the original solution in large excess since macrocycles are highly selective in their interaction with ions. The large data base for macrocycle-ion interaction allows for a judicious choice of the appropriate macrocycle for a desired analysis under a particular set of circumstances.

The Chemical Review 1985 Article referred to above gives data on certain carbon interactions with a number of compounds of the formula (A) given herein.

Formulae, cations the code for the ligand, log K data and the literature reference are given below in Table 3. It should be noted that the pK values vary with the medium used to determine the interaction and this should be checked by reference to the review article and the originial reference before reliance is placed on the specific numerical data given in Table 3 below.

However Table 3 indicates a range of specific macrocycle ligands and a range of cations with which they interact, to which ligands the present invention may be applied.

TABLE 3

| A | B | C | D | E | F | n | R | ligand | cation | log K | ref |
|---|---|---|---|---|---|---|---|---|---|---|---|
| NH | NH | O | – | – | O | -1 | | $A_2$12C4 | $Co^{2+}$ | 5.76 | 26 |
| | | | | | | | | | $Ni^{2+}$ | 5.91 | 26 |
| | | | | | | | | | $Cu^{2+}$ | 8.16 | 26 |
| | | | | | | | | | $Zn^{2+}$ | 6.22 | 26 |
| NH | NH | NH | – | – | NH | -1 | H | $A_4$12C4 | $Cu^{2+}$ | 24.8 | 28,31-34 |
| | | | | | | | | | $Zn^{2+}$ | 16.2 | 32 |
| | | | | | | | | | $Cd^{2+}$ | 14.3 | 32 |
| | | | | | | | | | $Hg^{2+}$ | 25.5 | 36 |
| | | | | | | | | | $Pb^{2+}$ | 15.9 | 32 |
| NH | NH | O | – | O | O | O | | 1,4-$A_2$15C5 | $Ni^{2+}$ | 5.05 | 26 |
| | | | | | | | | | $Cu^{2+}$ | 8.86 | 26 |
| | | | | | | | | | $Zn^{2+}$ | 5.04 | 26 |
| NH | O | NH | – | O | O | O | | 1,7-$A_2$15C5 (2.1) | $La^{3+}$ | 7.08 | 65 |
| | | | | | | | | | $La^{3+}$ | 14.4 | 65 |
| | | | | | | | | | $Pr^{3+}$ | 7.94 | 65 |
| | | | | | | | | | $Pr^{3+}$ | 14.5 | 65 |
| | | | | | | | | | $Ld^{3+}$ | 7.86 | 65 |
| | | | | | | | | | $Sm^{3+}$ | 7.00 | 65 |
| | | | | | | | | | $Sm^{3+}$ | 14.9 | 65 |
| | | | | | | | | | $Eu^{3+}$ | 8.59 | 65 |
| | | | | | | | | | $Eu^{3+}$ | 14.6 | 65 |
| | | | | | | | | | $Gd^{3+}$ | 7.67 | 65 |
| | | | | | | | | | $Tb^{3+}$ | 8.29 | 65 |
| | | | | | | | | | $Dy^{3+}$ | 8.96 | 65 |
| | | | | | | | | | $Dy^{3+}$ | 15.2 | 65 |
| | | | | | | | | | $Ho^{3+}$ | 8.81 | 65 |
| | | | | | | | | | $Er^{3+}$ | 8.70 | 65 |
| | | | | | | | | | $Er^{3+}$ | 14.8 | 65 |

| A | B | C | D | E | F | n | R | ligand | cation | log K | ref. |
|---|---|---|---|---|---|---|---|---|---|---|---|
| | | | | | | | | | $Y^{3+}$ | 8.66 | 65 |
| | | | | | | | | | $Tm^{3+}$ | 9.46 | 65 |
| | | | | | | | | | $Yb^{3+}$ | 15.4 | 65 |
| | | | | | | | | | $Co^{2+}$ | 5.05 | 77 |
| | | | | | | | | | $Co^{2+}$ | 5.22 | 26 |
| | | | | | | | | | $Ni^{2+}$ | 3.73 | 77 |
| | | | | | | | | | $Ni^{2+}$ | 4.05 | 26 |
| | | | | | | | | | $Cu^{2+}$ | 7.17 | 77 |
| | | | | | | | | | $Cu^{2+}$ | 8.15 | 26 |
| | | | | | | | | | $Zn^{2+}$ | 5.34 | 26 |
| | | | | | | | | | $Zn^{2+}$ | 5.19 | 77 |
| | | | | | | | | | $Ag^{+}$ | 5.85 | 77 |
| | | | | | | | | | $Cd^{2+}$ | 6.46 | 77 |
| S | O | O | – | O | O | O | | T15C5 | $Ag^{+}$ | 5.0 | 16 |
| | | | | | | | | | $Tl^{+}$ | 0.80 | 16 |
| | | | | | | | | | $Pb^{2+}$ | 1.65 | 16 |
| S | S | O | – | O | O | O | | 1,4-$T_2$15C5 | $Ag^{+}$ | 3.31 | 16 |
| | | | | | | | | | $Hg^{2+}$ | 5.1 | 16 |
| | | | | | | | | | $Tl^{+}$ | <0.6 | 16 |
| | | | | | | | | | $Pb^{2+}$ | 1.21 | 16 |
| S | O | S | – | O | O | O | | 1,7-$T_2$15C5 | $Ag^{+}$ | 2.7 | 16 |
| | | | | | | | | | $Hg^{2+}$ | 2.91 | 16 |
| | | | | | | | | | $Tl^{+}$ | <0.2 | 16 |
| | | | | | | | | | $Pb^{2+}$ | 1.62 | 16 |
| O | NH | S | – | S | NH | O | | 4,13-$A_2$-7, $T_2$15C5 | $Co^{2+}$ | 5.42 | 77,82,84 |
| | | | | | | | | | $Ni^{2+}$ | 7.98 | 77,82,84 |
| | | | | | | | | | $Cu^{2+}$ | 11.5 | 77,82,84 |
| | | | | | | | | | $Ag^{+}$ | 8.95 | 77,82 |
| | | | | | | | | | $Zn^{2+}$ | 5.09 | 77,82,84 |

| A | B | C | D | E | F | n | R | ligand | cation | log K | ref. |
|---|---|---|---|---|---|---|---|---|---|---|---|
| | | | | | | | | | $Cd^{2+}$ | 6.53 | 77,82 |
| | | | | | | | | | $Pb^{2+}$ | 5.67 | 77,82,84 |
| O | S | NH | - | NH | S | O | | 7,10-$A_2$-4, 13-$T_2$15C5 | $Co^{2+}$ | 5.22 | 82,83 |
| | | | | | | | | | $Ni^{2+}$ | 8.06 | 82,83 |
| | | | | | | | | | $Cu^{2+}$ | 13.26 | 82,83,85 |
| | | | | | | | | | $Ag^{+}$ | 9.91 | 82,83 |
| | | | | | | | | | $Zn^{2+}$ | 4.43 | 82,83 |
| | | | | | | | | | $Cd^{2+}$ | 7.13 | 82,83 |
| | | | | | | | | | $Pb^{2+}$ | 6.78 | 82,83,85 |
| NH | O | NH | O | O | O | 1 | | 1,7-$A_2$18C6 | $Co^{2+}$ | 3.26 | 26 |
| | | | | | | | | | $Ni^{2+}$ | 3.21 | 26 |
| | | | | | | | | | $Cu^{2+}$ | 7.40 | 26 |
| | | | | | | | | | $Zn^{2+}$ | 4.26 | 26 |
| NH | O | O | NH | O | O | 1 | | 1,10-$A_2$18C6 (2.2) | $Li^{+}$ | 1.07 | 87 |
| | | | | | | | | | $Na^{+}$ | 4.30 | 90 |
| | | | | | | | | | $K^{+}$ | 4.32 | 90 |
| | | | | | | | | | $Rb^{+}$ | 3.37 | 90 |
| | | | | | | | | | $Ca^{+}$ | 2.48 | 90 |
| | | | | | | | | | $Sr^{2+}$ | 2.57 | 99 |
| | | | | | | | | | $Ba^{2+}$ | 2.98 | 99 |
| | | | | | | | | | $La^{3+}$ | 6.18 | 87 |
| | | | | | | | | | $Co^{2+}$ | >3.5 | 87 |
| $NCH_3$ | O | O | $NCH_3$ | O | O | 1 | | $Me_2A_2$18C6 | $Na^{+}$ | 3.7 | 134 |
| | | | | | | | | | $K^{+}$ | 5.3 | 134 |
| | | | | | | | | | $Rb^{+}$ | 4.3 | 134 |
| | | | | | | | | | $Ca^{2+}$ | 4.4 | 134 |
| | | | | | | | | | $Sr^{2+}$ | 6.1 | 134 |
| | | | | | | | | | $Ba^{2+}$ | 6.7 | 134 |

| A | B | C | D | E | F | n | R | ligand | cation | log K | ref. |
|---|---|---|---|---|---|---|---|---|---|---|---|
| A | O | O | D | O | O | | | $(HOE)_2A_2$18C6 | $Na^+$ | <2 | 88 |
| A = D = N − $CH_2-CH_2-OH$ | | | | | | | | | $K^+$ | <2 | 88 |
| | | | | | | | | | $Mg^{2+}$ | <2 | 88 |
| | | | | | | | | | $Ca^{2+}$ | 4.08 | 88 |
| | | | | | | | | | $Cu^{2+}$ | 6.60 | 88 |
| | | | | | | | | | $Ag^+$ | 7.27 | 88 |
| | | | | | | | | | $Cd^{2+}$ | 7.96 | 88 |
| | | | | | | | | | $Pb^{2+}$ | 9.20 | 88 |
| NH | NH | O | NH | NH | O | 1 | | $A_4$18C6 | $Co^{2+}$ | 9.68 | 26 |
| | | | | | | | | | $Ni^{2+}$ | 12.49 | 26 |
| S | O | O | O | O | O | 1 | | T18C6 | $Na^+$ | 2.57 | 61 |
| | | | | | | | | | $K^+$ | 3.61 | 61 |
| | | | | | | | | | $Rb^+$ | 2.99 | 135a |
| | | | | | | | | | $Ba^{2+}$ | 3.4 | 61 |
| | | | | | | | | | $Ag^+$ | >5.5 | 61 |
| S | S | O | O | O | O | 1 | | 1,4-$T_2$18C6 | $Ag^+$ | 3.0 | 16 |
| | | | | | | | | | $Tl^+$ | 1.38 | 16 |
| | | | | | | | | | $Pb^{2+}$ | 2.63 | 16 |
| S | O | O | S | O | O | 1 | | 1,10-$T_2$18C6 | $K^+$ | 1.15 | 11 |
| | | | | | | | | | $Ag^+$ | 4.34 | 11,25 |
| | | | | | | | | | $Tl^+$ | 0.93 | 16 |
| | | | | | | | | | $Pb^{2+}$ | 3.13 | 16 |

**Claims**

1. A method of selectively and quantitatively removing and concentrating at least one selected ion present in low concentration with other ions present either at similar or much greater concentrations in a multiple ion solution which comprises:

(a) bringing a complexing agent for the selected ion(s) into contact with a sufficient determined quantity of the said multiple ion solution to remove and concentrate selected ion(s) from the multiple ion solution;

(b) removing the multiple ion solution from which the complexing agent has removed the selected ions from the complexing agent having the selected ion(s) complexed therewith;

(c) bringing the complexing agent complexed with selected ion(s) into contact with a smaller determined quantity of receiving liquid to break the complex and remove the selected and concentrated ion(s) from the complexing agent; and

(d) determining the concentration of selected ion(s) in the said receiving liquid from which the concentration of selected ion(s) in the multiple ion solution can be calculated.

2. A method of determining the heavy metal ion content of drinking water which is present in parts per billion which comprises flowing the drinking water in measured quantity through a column packed with silica covalently bonded to a complexing agent for the heavy metal ion(s), flowing a receiving liquid through the said column in lower quantity than the quantity of drinking water flowed through the said column to break the complex and take the liberated ions into solution, analyzing the solution to determine the amount of heavy metal ion(s) therein, and calculating therefrom the concentration of heavy metal ions in the drinking water.

3. A process of determining the heavy metal ion content of drinking water which is present in parts per billion which comprises flowing the drinking water in measured quantity through a column packed with a complexing agent which is a macrocyclic compound having at least four -A-$CH_2$-$CH_2$-A- groups in which A represents -O-, -O-$CH_2$-, -S-, -S-$CH_2$, -N-R- or -N-(R)-$CH_2$- in which R represents a hydrogen atom, a lower alkyl group or a benzyl group and which has a hydrocarbon side chain having an end group

```
 CH3
 |
-Si-O-  covalently bonded to silica
 |
 X
```

in which X represents a lower alkyl, benzyl, phenyl, methoxy, or ethoxy group or a halogen atom, or an Si≡ group, flowing a receiving liquid through the said column in lower quantity than the quantity of drinking water flowed through the said column to break the complex and take the liberated ions into solution, analyzing the solution to determine the amount of heavy metal ion(s) therein, and calculating therefrom the concentration of heavy metal ions in the said drinking water.

4. A method of achieving the selective and quantitative removal of a selected ion or group of ions present at low concentrations from a plurality of ions in a multiple ion solution in which the other ions may be present at much higher concentrations by bringing a known amount of the solution into contact with a composition of matter comprising silica covalently bonded to a macrocyclic compound having the formula:

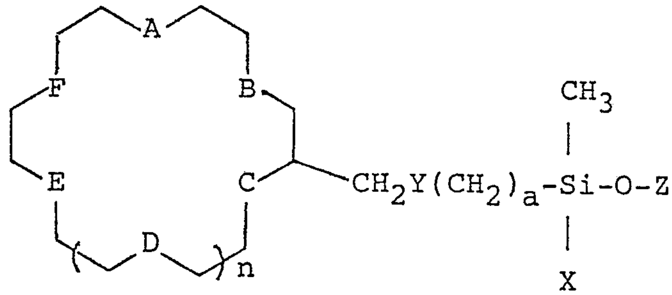

(A)

in which A to F each represent an oxygen atom or an O-$CH_2$ group or a sulphur atom or an S-$CH_2$ group or an N-R group or an N(R)$CH_2$ group in which R represents a hydrogen atom or an alkyl group or an aryl group, n is -1, 0, 1, 2, 3 or 4, X represents an alkyl group or an aryl group, or an alkoxy group, or a halogen atom, or an O-silica group, or an O-silica gel group, Y represents an oxygen atom or a $CH_2$ group, a is an integer from 1 to 18, and Z represents silica or silica gel;

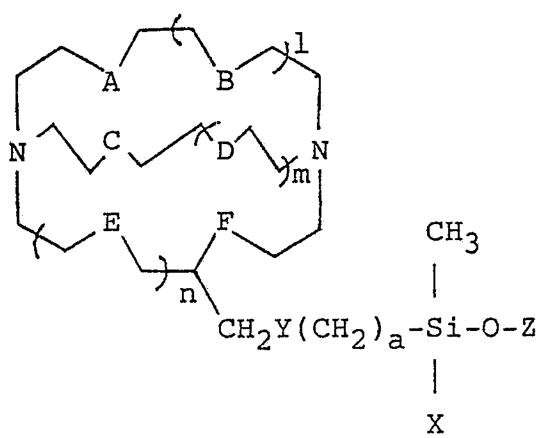

(B)

in which A to F each represent an oxygen atom or a sulphur atom or an N-R group in which R represents a hydrogen atom or an alkyl group or an aryl group, l, m and n are each 0, 1 or 2, X represents an alkyl group, or an aryl group, or an alkoxy group, or a halogen atom or an O-silica group or an O-silica gel group, Y represents an oxygen atom or a $CH_2$ group, a is an integer from 1 to 18, and Z represents silica or silica gel;

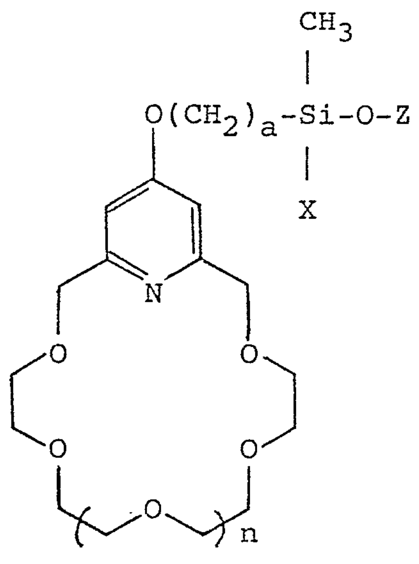

(C)

in which n is 0, 1 or 2, a is an integer from 1 to 18, X represents an alkyl group, or an aryl group, or an alkoxy group or a halogen atom or an O-silica group or an O-silica gel group, and Z represents silica or silica gel; or

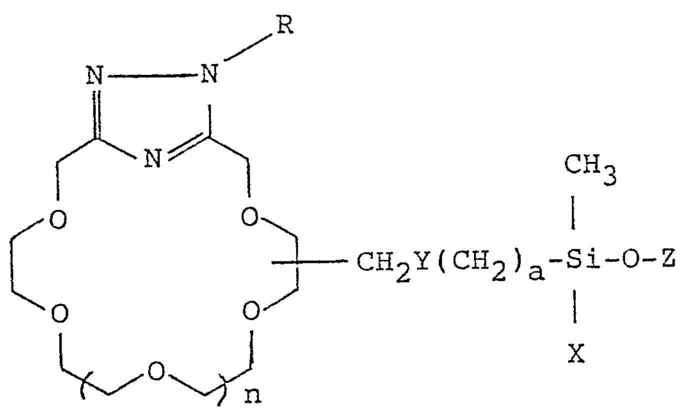

(D)

in which a is an integer from 1 to 18, Y represents an oxygen atom or a $CH_2$ group, R represents a hydrogen atom or any alkyl group or an aryl group, and X represents an alkyl group, or an aryl group, or an alkoxy group, or a halogen atom or an O-silica group or an O-silica gel group, and Z represents silica or silica gel.

5. A method as claimed in Claim 4 in which the complexing agent is of formula A and A to F each represent an oxygen atom or a sulphur atom or an N-R group in which R represents a hydrogen atom or an alkyl group or a benzyl group, or of formula B and l, m and n are each 1, or of formula D and R represents an alkyl group.

6. A method as claimed in Claim 4 in which the complexing agent is of formula A and A, C, D and F each represent an oxygen atom and B and E each represent an N-R group, or A to F each represent an oxygen atom.

7. A method as claimed in Claim 4, 5 or 6 in which the at least one selected ion which has been complexed with the said macrocyclic compound is concentrated by eluting the said selected ion with a receiving liquid in much smaller volume than the volume of liquid from which it has been complexed.

8. A method as claimed in Claim 7 in which the concentration of the selected ion in the receiving liquid is determined, thereby enabling the concentration in the drinking water to be calculated.

9. A method as claimed in Claim 1 or Claim 2 of separating a selected ion from a plurality of other ions in a multiple ion solution characterized by flowing the multiple ion solution through a column packed with plain silica gel.

10. A method as claimed in Claim 1 or Claim 2 or Claim 3 in which the complexing agent is the composition of matter specified in any one of Claims 4, 5 or 6.

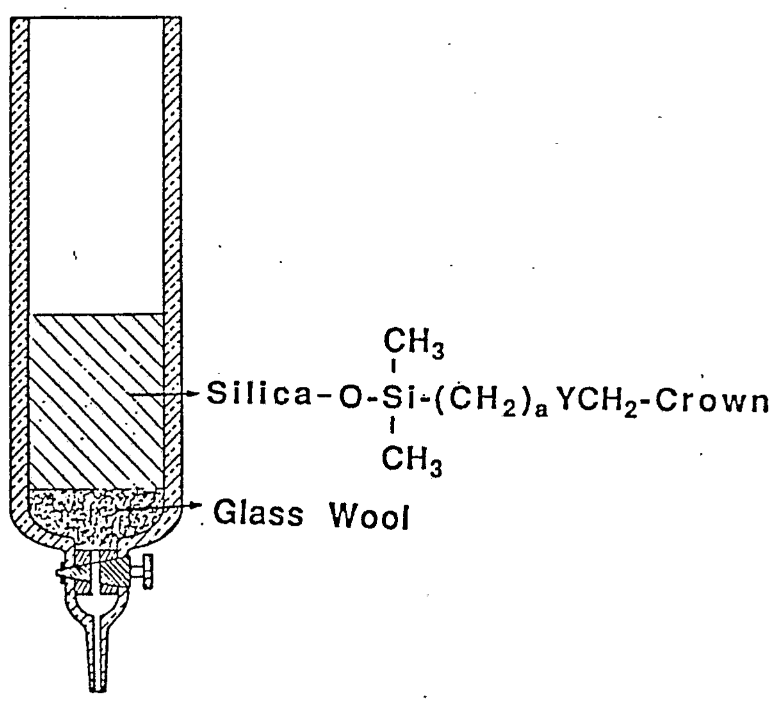
Silica-O-Si-(CH2)a YCH2-Crown
Glass Wool

European Patent Office

# EUROPEAN SEARCH REPORT

Application number

EP 88308159.8

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | EP - A1 - 0 073 261 (VERKAUFSFORSCHUNGSZENTRUM KARLSRUHE GMBH)<br>* Page 8 - page 9, second paragraph; claims *<br>-- | 1,4,9,10 | G 01 N 33/18<br>B 01 J 45/00 |
| P,Y | EP - A2 - 0 237 301 (J.T.BAKER CHEMICAL CO.)<br>* Claims *<br>-- | 1,3 | |
| A | EP - A1 - 0 018 102 (THE BRITISH PETROLEUM COMPANY LIMITED)<br>* Claims *<br>---- | 1 | |
| | | | TECHNICAL FIELDS SEARCHED (Int. Cl.4) |
| | | | G 01 N<br>B 01 J<br>G 21 F 9/00 |

The present search report has been drawn up for all claims

| Place of search | Date of completion of the search | Examiner |
|---|---|---|
| VIENNA | 15-12-1988 | TENGLER |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document